# DEMANDE DE BREVET EUROPEEN

(11) **EP 1 186 280 A1**
(43) Date de publication de la demande: **13.03.2002**
(21) Numéro de dépôt: 01402348.5
(22) Date de dépôt: 12.09.2001
(51) Int. Cl.: A61F 9/007

(54) **Attelle pour la chirurgie des voies lacrymales**

(30) Priorité: 12.09.2000 FR 0011568
(71) Demandeur: F.C.I. (FRANCE CHIRURGIE INSTRUMENTATION), F-92134 Issy-Les Moulineaux (FR)
(72) Inventeur: Bernard, Jean-Antoine, 75116 Paris (FR); Klap, Patrick, 75116 Paris (FR)
(74) Mandataire: Faber, Jean-Paul

(57) **Abrégé**

Attelle pour la chirurgie des voies lacrymales caractérisée en ce qu'elle est constituée d'une plaquette en matière souple (1) formant deux branches (2 et 3) disposées suivant un angle ouvert, l'une des branches (2), le long de son bord situé du côté convexe, comportant un bourrelet (4) en saillie sur l'une de ses faces.

## Description

La présente invention vise une attelle pour la chirurgie des voies lacrymales.

L'invention vise notamment une attelle plus particulièrement utilisable pour la dacryocystorhinostomie par voie endonasale, opération consistant à réaliser un abouchement du sac lacrymal dans les fosses nasales, suite à une obstruction des voies lacrymales.

Après avoir pratiqué une telle opération, afin de réaliser l'hémostase, on insère dans la fosse nasale des mèches. Ces mèches sont inconfortables à supporter pour le patient et sont souvent source d'infection.

L'un des buts de la présente invention est de réaliser une attelle simple à fabriquer, d'une utilisation aisée pour le chirurgien, qui assure une bonne cicatrisation dans de meilleures conditions de confort pour le patient : respiration nasale préservée, absence d'infections postopératoires et d'odeurs nauséabondes.

L'attelle, selon l'invention, est caractérisée en ce qu'elle est constituée d'une plaquette en matière souple formant deux branches disposées suivant un angle ouvert, l'une des branches comportant, le long de son bord situé du côté convexe, un bourrelet en saillie sur l'une de ses faces.

Bien entendu, il est prévu une attelle correspondant au côté gauche et une correspondant au côté droit.

L'attelle, après l'opération, est engagée dans la fosse nasale de manière que le bourrelet vienne se situer en regard du sac lacrymal, ledit bourrelet étant ouvert à ses extrémités ; il permet au patient de respirer librement ce qui est plus confortable que les mèches généralement utilisées, mèches qui sont souvent une source d'infections postopératoires et d'odeurs nauséabondes.

De préférence, le bourrelet est légèrement élastique.

Suivant un détail constructif, le bourrelet affecte la forme d'un onglet cylindrique.

Enfin, suivant une dernière caractéristique, le bord du bourrelet, opposé à celui solidaire du bord de la branche correspondante est libre, un petit espace étant ménagé entre ledit bord libre et la face correspondante de ladite branche.

L'invention va maintenant être décrite avec plus de détails en se référant à un mode de réalisation particulier donné à titre d'exemple seulement et représenté aux dessins annexés, dans lesquels :

Figure 1 et figure 2 sont des vues en perspective d'une attelle, selon l'invention, l'une étant destinée à la fosse nasale gauche et l'autre à la fosse nasale droite.

Figure 3 est une vue en coupe suivant la ligne 3-3 de la figure 1.

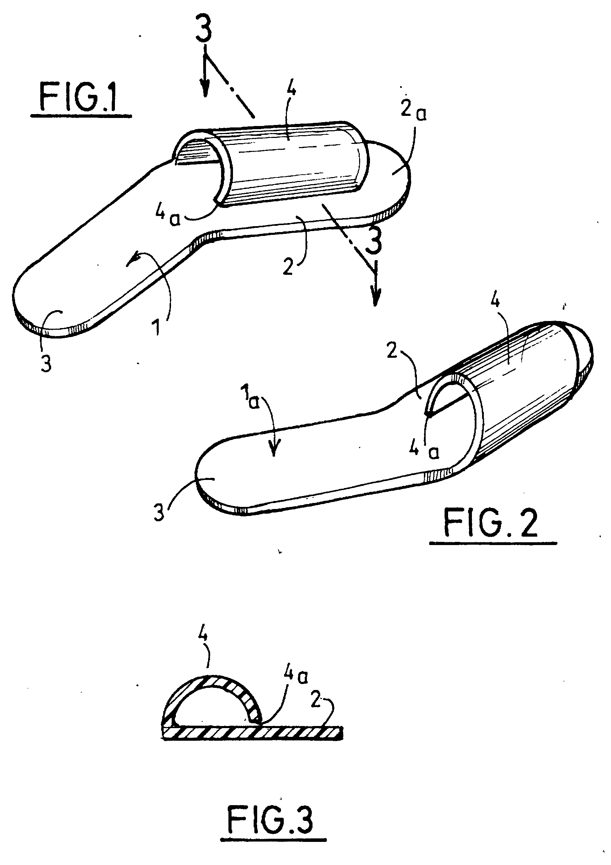

L'attelle représentée aux figures 1 et 2 est constituée d'une plaquette en matière souple 1 présentant deux branches 2 et 3 formant entre elles un angle ouvert et dont les extrémités sont légèrement arrondies.

La branche 2, le long de son bord 2a et du côté convexe de la plaquette, est solidaire d'un bourrelet 4 légèrement élastique, celui-ci ayant la forme générale d'un onglet cylindrique dont les deux plans délimitant ledit onglet sont situés dans un même plan.

Le bord 4a du bourrelet 4 opposé à celui solidaire du bord 2a est libre et est en position normale situé à quelques millimètres de la surface de la branche 2.

L'attelle de la figure 1 est destinée à être utilisée du côté gauche ; à la figure 2, on a représenté une attelle 1a destinée au côté droit, celle-ci étant réalisée de la même manière que celle qui a été décrite pour la figure 1.

Après une opération destinée à soigner une obstruction des voies lacrymales, par exemple, à la suite d'une dacryocystite, on insère dans la fosse nasale, du côté opéré, l'attelle selon l'invention de manière que le bourrelet 4 vienne se situer en regard du sac lacrymal. Celui-ci, compte tenu de son élasticité, permet de maintenir écartées les parois de la fosse nasale autorisant ainsi le libre passage des larmes. De plus, le patient peut respirer librement ce qui est plus confortable.

Afin que l'attelle qui peut être maintenue plusieurs jours ne bouge pas, on peut fixer celle-ci par un ou plusieurs fils traversant la plaquette et la cloison nasale.

Bien entendu, l'invention n'est pas limitée au mode de réalisation qui vient d'être décrit et représenté. On pourra y apporter de nombreuses modifications de détail sans sortir pour cela du cadre de l'invention.

## Revendications

1. Attelle pour la chirurgie des voies lacrymales **caractérisée en ce qu'**elle est constituée d'une plaquette en matière souple (1) formant deux branches (2 et 3) disposées suivant un angle ouvert, l'une des branches (2) comportant, le long de son bord situé du côté convexe, un bourrelet (4) en saillie sur l'une de ses faces.

2. Attelle pour la chirurgie des voies lacrymales, selon la revendication 1, **caractérisée en ce que** le bourrelet (4) est élastique.

3. Attelle pour la chirurgie des voies lacrymales, selon la revendication 1, **caractérisée en ce que** le bourrelet (4) affecte la forme d'un onglet cylindrique.

4. Attelle pour la chirurgie des voies lacrymales, selon la revendication 1, **caractérisée en ce que** le bord du bourrelet (4), opposé à celui solidaire du bord de la branche (2) correspondante, est libre, un petit espace étant ménagé entre ledit bord libre et la face correspondante de ladite branche.
